# FASCICULE DE BREVET EUROPEEN

(11) **EP 1 254 253 B1**
(45) Date de publication et mention de la délivrance du brevet: **12.07.2006**
(21) Numéro de dépôt: 00962582.3
(22) Date de dépôt: 08.09.2000
(51) Int. Cl.: C12Q 1/68, A61K 38/00

(54) **OLIGONUCLEOTIDES MONOCATENAIRES, SONDES, AMORCES ET PROCEDE DE DETECTION DES SPIROCHETES**
EINZELSTRÄNGIGE OLIGONUKLEOTIDE, SONDEN, PRIMER UND EIN VEFAHREN ZUR DETEKTION VON SPIROCHAETEN
SINGLE STRANDED OLIGONUCLEOTIDES, PROBES, PRIMERS AND METHOD FOR DETECTING SPIROCHETES

(30) Priorité: 10.09.1999 FR 9911493
(43) Date de publication de la demande: 06.11.2002
(73) Titulaire: BIOMERIEUX SA, 69280 Marcy L'Etoile (FR)
(72) Inventeur: RAOULT, Didier, F-13008 Marseille (FR); DRANCOURT, Michel, F-13012 Marseille (FR)
(74) Mandataire: Domange, Maxime
(86) Numéro de dépôt international: PCT/FR2000/002481
(87) Numéro de publication internationale: WO 2001/020028

(56) Documents cités:
- WO-A-96/23071
- WO-A-97/11162
- WO-A-98/44130
- WO-A-98/58943
- WO-A-98/59034
- US-A- 5 786 147
- DATABASE EMHUM6 [en ligne] EMBL; XP002140071 & ALAIBAC ET AL.: "High incidence of a T nucleotide at the second position of codon 97 in Vdelta2-Jdelta1 junctional sequences of human normal skin gamma-delta T-cells" MOLECULAR IMMUNOLOGY, vol. 33, 1996, pages 1035-1038,
- ALEKSHUN M ET AL: "Molecular cloning and characterization of Borrelia burgdorferi rpoB" GENE: AN INTERNATIONAL JOURNAL ON GENES AND GENOMES,GB,ELSEVIER SCIENCE PUBLISHERS, BARKING, vol. 186, no. 2, 28 février 1997 (1997-02-28), pages 227-235, XP004093305 ISSN: 0378-1119 cité dans la demande
- MOLLET C ET AL: "rpoB sequence analysis as a novel basis for bacterial identification." MOLECULAR MICROBIOLOGY, (1997 DEC) 26 (5) 1005-11., XP000920777
- RENESTO PATRICIA ET AL: "rpoB gene analysis as a novel strategy for identification of spirochetes from the genera Borrelia, Treponema, and Leptospira." JOURNAL OF CLINICAL MICROBIOLOGY, vol. 38, no. 6, juin 2000 (2000-06), pages 2200-2203, XP002155811 ISSN: 0095-1137

## Description

La présente invention concerne le domaine des techniques de détection et/ou d'amplification et de séquençage, à l'aide de sondes ou d'amorces oligonucléotidiques, et l'application de ces sondes ou amorces pour détecter la présence ou identifier les bactéries de l'ordre des *Spirochaetales* (spirochètes).

Dans le cadre de certains tests de diagnostic, notamment lors d'une recherche d'infection humaine ou animale dans le système nerveux ou d'une recherche après piqûre d'arthropode par exemple, il est souvent nécessaire d'obtenir une réponse rapide concernant la présence de bactéries, et plus précisément de spirochètes, dans un échantillon. Toutefois, les techniques couramment développées, telles qu'un examen direct après coloration de Gram ou une mise en culture, sont souvent mises en échec par l'absence de prise de colorant de Gram et l'absence de croissance dans les milieux de culture.

Dans le cas des spirochètes, qui constituent une famille de bactéries regroupant les espèces *Leptospira, Borrelia, Treponema* et *Serpulina* et étant responsables de maladies infectieuses, telles que la méningoencéphalite, la mise en culture n'est pas possible. L'identification des spirochètes n'a donc pas encore été résolue.

Etant donné l'accroissement de ces maladies infectieuses ces vingt dernières années et les conséquences dramatiques de ces états pathologiques infectieux, il est nécessaire de mettre au point une méthode rapide et spécifique de détection des pathogènes infectieux que sont les spirochètes.

Une solution possible, permettant de palier à l'impossibilité d'une mise en culture des bactéries, réside dans l'utilisation des technologies relatives aux acides nucléiques et au matériel génétique, notamment des méthodologies PCR (Polymerase Chain Reaction), afin de déterminer si un gène, une partie de gène ou une séquence nucléotidique est présent chez un organisme vivant, un extrait cellulaire de cet organisme ou un échantillon. Etant donné que tout gène ou partie de gène est caractérisé par une séquence spécifique de bases nucléotidiques, il est par conséquent possible de rechercher directement la présence de tout ou partie de ladite séquence spécifique au sein d'un échantillon contenant un mélange de polynucléotides.

Différents types de méthodes de détection des acides nucléiques sont décrits dans la littérature. Ces méthodes reposent sur les propriétés d'appariement purine-pyrimidine des brins complémentaires d'acides nucléiques dans les duplex ADN-ADN et ARN-ARN. Ce processus d'appariement s'effectue par l'établissement de liaisons hydrogène entre les bases adénine-thymine (A-T) et guanine-cytosine (G-C) de l'ADN double brin. Des paires de bases adénine-uracile (A-U) peuvent également se former par liaison hydrogène dans les duplex ADN-ARN ou ARN-ARN. L'appariement de brins d'acide nucléique pour la détermination de la présence ou de l'absence d'une molécule d'acide nucléique donnée est communément appelé " hybridation d'acides nucléiques " ou simplement " hybridation ".

Un exemple de méthodologie PCR comprend la détermination d'une séquence sur la base de l'ARNr 16S. Toutefois, cette méthode présente des limites liées aux problèmes potentiels de contaminations qui gênent le diagnostic.

Pour pallier à cet inconvénient, de nouveaux marqueurs génétiques permettant la détection spécifique de bactéries appartenant à l'ordre des spirochètes dans tout échantillon, sans étape préalable de culture bactérienne, ont été trouvés. Ces nouveaux marqueurs, qui sont des oligonucléotides, constituent un objet de l'invention.

La détermination de ces nouveaux marqueurs repose sur l'utilisation de séquences spécifiquement définies dans le gène *rpoB* des spirochètes codant pour la sous-unité β de l'ARN polymérase bactérienne. En effet, on a trouvé sur l'ADN codant pour la sous-unité β de l'ARN polymérase bactérienne, des zones qui sont variables selon les familles bactériennes, mais qui apparaissent conservées parmi l'ordre des spirochètes, ce qui permet de discriminer cette famille bactérienne parmi d'autres familles bactériennes. L'observation selon laquelle il existe dans lesdites zones conservées des variations mineures de séquences entre certaines espèces de spirochètes a permis de mettre au point ces marqueurs spécifiques de l'ordre des spirochètes.

Selon Lazcano et al. [J. Mol. Evol. (1988) 27:365-376], les ARN polymérases sont divisées en deux groupes selon leur origine, l'un constitué par les ARN polymérases virales ARN- ou ADN-dépendantes, et l'autre constitué par les ARN polymérases ADN-dépendantes d'origine eucaryote ou procaryote (archaebactéries et eubactéries). Les ARN polymérases ADN-dépendantes eubactériennes sont caractérisées par une constitution multimérique simple et conservée notée " core enzyme ", représentée par *αββ',* ou " holoenzyme " représentée par αββ'σ[Yura and Ishihama, Ann. Rev. Genet. (1979) 13 : 59-97].

De nombreux travaux ont mis en évidence le rôle fonctionnel, au sein du complexe enzymatique multimérique, de la sous-unité β de l'ARN polymérase eubactérienne. Les ARN polymérases archaebactérienne et eucaryote présentent, pour leur part, une structure plus complexe pouvant atteindre une dizaine, voire une trentaine de sous-unités [Pühler et al . Proc. Natl. Acad. Sci. USA (1989) 86 :4569-4573].

Les gènes qui codent pour les différentes sous-unités *α,ββ'σ* de l'ARN polymérase ADN-dépendante chez les eubactéries, respectivement les gènes *rpoA, rpoB, rpoC* et *rpoD,* sont classés en différents groupes comprenant des gènes codant pour des protéines constitutives des sous-unités ribosomiques ou pour des enzymes impliquées dans la réplication et la réparation du génome [Yura and Yshihma, Ann. Rev. Genet. (1979) 13 :59-97]. Certains auteurs ont montré que les séquences nucléiques des gènes *rpoB* et *rpoC* pouvaient être utilisées afin de construire des arbres phylogénétiques [Rowland et al., Biochem. Soc. Trans. (1992) 21:40s] permettant de séparer les différents embranchements et sous-embranchements parmi les règnes du vivant.

Il a été décrit des sondes pour la détection de bactéries Borrelia burgdorferi et Treponema pallidum dans WO 98 59034 et WO 98 58943.

D'autre part, dans MOLET et al (molecular microbiology 1997, 26 (5), 1005-11), il est décrit l'utilisation du gène rpoB comme marqueur génétique pour l'identification de bactéries de la famille des entérobactéries.

Avant d'exposer plus en détail l'invention, différents termes, utilisés dans la description et les revendications, sont définis ci-après :
- par " acide nucléique extrait de bactéries " on entend soit l'acide nucléique total, soit l'ADN génomique, soit les ARN messagers, soit encore l'ADN obtenu à partir de la transcription inverse des ARN messagers ;
- un " fragment nucléotidique " ou un " oligonucléotide " sont deux termes synonymes désignant un enchaînement de motifs nucléotidiques de type ADN ou ARN caractérisé par une séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminées de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 10, et en particulier au moins 12 motifs nucléotidiques et peut être obtenu à partir d'une molécule d'acide nucléique naturelle et/ou par recombinaison génétique et/ou par synthèse chimique,
- « un motif nucléotidique » est dérivé d'un monomère qui peut être un nucléotide naturel d'acide nucléique dont les éléments constitutifs sont un sucre, un groupement phosphate et une base azotée choisie parmi l'adénine, la guanine, l'uracile, la cytosine, la thymine ; ou bien le monomère est un nucléotide modifié dans l'un au moins des trois éléments constitutifs précédents ; à titre d'exemple, la modification peut intervenir soit au niveau des bases, avec des bases modifiées telles que l'inosine qui peut s'hybrider avec toute base A, T, U, C ou G, la méthyl-5-désoxycytidine, la désoxyuridine, la diméthylamino-5-désoxyuridine ou toute autre base modifiée capable d'hybridation, soit au niveau du sucre, par exemple le remplacement d'au moins un désoxyribose par un polyamide [PE Nielsen et al., Science, (1991) 254:1497-1500], soit encore au niveau du groupement phosphate, par exemple par remplacement par des esters choisis notamment parmi les diphosphates, les alkyl- et arylphosphonates et les phosporothioates,
- par " séquence informationnelle ", on entend toute suite ordonnée de motifs de type nucléotidique, dont la nature chimique et l'ordre dans un sens de référence constituent une information analogue à celle donnée par la séquence des acides nucléiques naturels,
- par " hybridation ", on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la " stringence ", c'est-à-dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65 °C, en particulier entre 35 et 65°C dans une solution saline à une concentration d'environ 0,8 à 1M,
- une " sonde " est un fragment nucléotidique comprenant par exemple de 10 à 100 motifs nucléotidiques, notamment de 12 à 35 motifs nucléotidiques, possédant une spécificité d'hybridation dans des conditions déterminées pour former un complexe d'hybridation avec un acide nucléique ayant, dans le cas présent, une séquence nucléotidique comprise soit dans un ARN messager, soit dans un ADN obtenu par transcription inverse dudit ARN messager, produit de transcription; une sonde peut être utilisée à des fins de diagnostic (notamment sondes de capture ou de détection ) ou à des fins de thérapie,
- une " sonde de capture " est immobilisée ou immobilisable sur un support solide par tout moyen approprié, par exemple par covalence, par adsorption, ou par synthèse directe sur un solide. Des exemples de supports comprennent les plaques de microtitration et les puces à ADN,
- une " sonde de détection " peut être marquée au moyen d'un agent marqueur choisi par exemple parmi les isotopes radioactifs, les enzymes, en particulier les enzymes susceptibles d'agir sur un substrat chromogène, fluorigène ou luminescent (notamment une peroxydase ou une phosphatase alcaline), les composés chimiques chromophores, les composés chromogènes, fluorigènes ou luminescents, les analogues de bases nucléotidiques et les ligands tels que la biotine,
- une " sonde d'espèce " est une sonde permettant l'identification de l'espèce d'une bactérie,
- une " sonde de genre " est une sonde permettant l'identification du genre d'une bactérie,
- une " amorce " est une sonde comprenant par exemple de 10 à 100 motifs nucléotidiques et possédant une spécificité d'hybridation dans des conditions déterminées pour l'initiation d'une polymérisation enzymatique, par exemple dans une technique d'amplification telle que la PCR, dans un procédé de séquençage, dans une méthode de transcription, etc.

Un premier objet de la présente invention est un oligonucléotide monocaténaire choisi parmi les oligonucléotides ayant une séquence d'au moins 12 motifs nucléotidiques consécutifs incluse dans l'une des séquences SEQ ID N°1 à SEQ ID N°4 décrites dans le listage de séquences en fin de description et parmi les oligonucléotides complémentaires de ces oligonucléotides.

Dans chacune des séquences SEQ ID n° 1 à 4, le nucléotide « N » mentionné dans le listage de séquences en fin de description représente un nucléotide identique ou différent à chacune des 3 positions où il apparaît et peut représenter l'inosine ou un mélange équimolaire de 4 nucléotides différents choisis parmi A, T, C et G ou le cas échéant A, U, C et G.

Lorsque « N » représente undit mélange équimolaire de nucléotides à une position donnée, cela signifie que le nucléotide à ladite position donnée représente indifféremment A, T, C ou G (ou respectivement le cas échéant A, U, C ou G) et que l'oligonucléotide selon l'invention est constitué plus exactement d'un mélange équimolaire de 4 groupes d'oligonucléotides dans chacun desquels groupes « N » a une signification différente à ladite position donnée et représente respectivement dans chacun des 4 groupes A, T, C et G.

Dans chacune des séquences SED ID n° 1 à 4, lorsque dans les 3 positions où un nucléotide « N » apparaît, N représente undit mélange équimolaire des 4 nucléotides A, T, C et G (ou N le cas échéant A, U, C et G), l'oligonucléotide comprenant ladite séquence représente donc un mélange de 4³ (64) oligonucléotides.

Comme mentionné dans les définitions, l'oligonucléotide selon l'invention peut être sous forme d'un oligodésoxyribonucléotide (ADN) ou d'un oligoribonucléotide (ARN) pour lesquels dans ce cas « T » est remplacé par « U ».

En particulier, un oligonucléotide selon la présente invention possède au moins 12 motifs tels que décrits ci-dessus et au plus 50 motifs. Plus particulièrement, un oligonucléotide selon la présente invention possède de 12 à 35 motifs.

Un oligonucléotide préféré a une séquence choisie parmi les séquences SEQ ID N° 1 à 4, ou les séquences complémentaires.

La séquence SEQ ID N°1, qui est la séquence de l'amorce appelée amorce n°1, possède 20 acides nucléiques. La séquence SEQ ID N°2, qui est la séquence de l'amorce appelée amorce n° 2, possède 21 acides nucléiques. La séquence SEQ ID N°3, qui est la séquence de l'amorce appelée amorce n° 3, possède 20 acides nucléiques. Enfin, la séquence SEQ ID N°4, qui est la séquence de l'amorce appelée amorce n°4, possède 17 acides nucléiques.

Dans la nomenclature faisant référence au gène *rpoB* chez E.COLI, les premiers nucléotides des séquences SEQ ID n° 1 à 4 correspondent aux positions. suivantes :
- SEQ ID n° 1 : 1 730,
- SEQ ID n° 2 : 2 900,
- SEQ ID n° 3 : 3 700,
- SEQ ID n° 4 : 3 850.

Les inventeurs ont découvert et mis en évidence que lesdites séquences SEQ ID n° 1 à 4, telles que définies ci-dessus, sont non seulement consensuelles entre les spirochètes, mais qu'elles sont en outre spécifiques de la famille des spirochètes et encadrent un fragment du gène *rpoB* comprenant une zone très variable dont la séquence est spécifique de l'espèce et du genre serovar dans une espèce de *spirochète.*

En effet, aux positions correspondant à celles des nucléotides « N » dans les séquences SEQ ID n° 1 à 4, on trouve des nucléotides variables dans les séquences cible complémentaires en fonction de l'espèce de la bactérie considérée, mais les autres nucléotides sont conservées dans toutes les espèces de bactéries de la famille des spirochètes. Et, du fait que « N » représente l'inosine qui peut s'hybrider avec toute base, ou un mélange équimolaire des 4 bases A, T, C et G, un oligonucléotide ou un mélange d'oligonucléotides selon l'invention comprend toujours un oligonucléotide pouvant s'hybrider avec une séquence cible complémentaire d'une bactérie spirochète.

En outre, comme les séquences SEQ ID n° 1 à 4 encadrent des zones variables dont les séquences sont spécifiques pour chaque espèce de bactérie de la famille des spirochètes, les séquences SEQ ID n° 1 à 4 permettent non seulement de préparer les sondes spécifiques de la famille des spirochètes, mais aussi de détecter et identifier l'espèce de ladite bactérie par amplification PCR en utilisant lesdites séquences comme amorces.

Dans un mode de réalisation des oligonucléotides selon l'invention, « N » représente dans toutes les positions une inosine.

Dans un autre mode de réalisation, l'oligonucléotide selon l'invention représente un mélange d'oligonucléotides (notamment 4^{P} oligonucléotides) comprenant des séquences incluses dans l'une des séquences SEQ ID n° 1 à 4, dans lesquelles tous les nucléotides A, T, C et G sont représentés à chacune desdites positions où « N » apparaît (notamment p positions, p représentant un nombre entier).

Les séquences SEQ ID N° 1 à 4 peuvent être préparées par synthèse chimique en utilisant les techniques bien connues de l'homme du métier et décrites par exemple dans l'article de Itakura K. et al. [(1984) Annu. Rev. Biochem. 53:323].

Une première application d'un oligonucléotide de l'invention est son utilisation comme sonde pour la détection, dans un échantillon biologique, de bactéries appartenant à l'ordre des spirochètes qui comprend une séquence nucléotidique d'au moins 12 motifs nucléotidiques consécutifs incluse dans l'une des séquences SEQ ID N° 1 à SEQ ID N°4, et leurs séquences complémentaires. Dans la suite de la description, une telle sonde de l'invention sera appelée « sonde de genre ».

Les sondes selon l'invention peuvent être utilisées, à des fins de diagnostic, dans la recherche de la présence ou de l'absence d'un acide nucléique cible dans un échantillon, selon toutes les techniques d'hybridation connues et notamment les techniques de dépôt ponctuel sur filtre, dites " DOT-BLOT " [Maniatis et al. (1982) Molecular Cloning, Cold Spring Harbor], les techniques de transfert d'ADN dites " SOUTHERN BLOT " [Southern. E.M., J. Mol. Biol. (1975) 98:503], les techniques de transfert d'ARN dites " NORTHERN BLOT ", ou les techniques dites " sandwich " [Dunn A.R., Hassel J.A. (1977) Cell 12:23]. On utilise en particulier la technique " sandwich ", avec une sonde de capture et/ou une sonde de détection, lesdites sondes étant capables de s'hybrider avec deux régions différentes de l'acide nucléique cible, et l'une au moins desdites sondes (généralement la sonde de détection) étant capable de s'hybrider avec une région de la cible qui est spécifique de l'espèce ou du groupe d'espèces recherché, étant entendu que la sonde de capture et la sonde de détection doivent avoir des séquences nucléotidiques au moins partiellement différentes.

L'acide nucléique à détecter (cible) peut être de l'ADN ou de l'ARN (l'un ou l'autre éventuellement obtenu après amplification par PCR). Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80 °C.

Pour mettre en oeuvre les techniques d'hybridation précitées, et en particulier les techniques " sandwich ", une sonde de l'invention, appelée sonde de capture, est immobilisée sur un support solide, et une autre sonde de l'invention, appelée sonde de détection, est marquée avec un agent.

Les exemples de support et d'agent marqueur sont tels que définis précédemment.

Un autre objet de l'invention est un procédé de détermination de la présence ou de l'absence d'au moins un spirochète, dans un échantillon contenant ou susceptible de contenir des acides nucléiques d'au moins une telle bactérie, comprenant les étapes consistant à mettre en contact ledit échantillon avec au moins une sonde de genre de l'invention, puis à déterminer de façon connue en soi la formation ou l'absence de formation d'un complexe d'hybridation entre ladite sonde et l'acide nucléique de l'échantillon.

Des exemples de détection de la formation ou l'absence de formation d'un complexe d'hybridation entre ladite sonde et l'acide nucléique comprennent les techniques décrites ci-dessus, à savoir les techniques "DOT-BLOT", "SOUTHERN-BLOT" et "sandwich".

Selon une mise en oeuvre particulière de ce procédé pour la détermination de la présence ou l'absence d'une espèce ou d'un groupe d'espèces de spirochète, on utilise d'une part une sonde de genre de l'invention et d'autre part une sonde d'espèce de l'invention, étant entendu que lesdites sondes de genre et d'espèce sont capables de s'hybrider avec des régions non chevauchantes d'un acide nucléique correspondant au gène *rpoB* des spirochètes.

De manière avantageuse, la sonde de genre est immobilisée sur un support solide, et la sonde d'espèce est marquée avec un agent marqueur.

La présente invention a aussi pour objet l'application du procédé de l'invention pour déterminer la présence d'une espèce de spirochète déterminée.

En effet, les résultats des recherches, mettant en évidence les alignements de séquences conservées chez les espèces de spirochètes, selon la méthode d'alignement CLUSTAL [Higgins D.G. & Sharp P.M. (1989) Gene 73:237-244], lesdites séquences ayant 1170 bases situées entre les positions 1730 et 2900 du gène *rpoB* en faisant référence à la numérotation du gène *rpoB* de *Escherichia coli* ATCC 25290, permettent de détecter la présence ou l'absence d'au moins une bactérie quelconque de l'ordre des spirochètes. L'utilisation de sondes contenant des zones mutées pour une espèce particulière (par rapport à l'espèce de référence, ici *E*. *coli*)*,* rend possible la détection directe d'une telle espèce. Dans les techniques d'hybridation sandwich, utilisant deux sondes en combinaison (sonde de capture et sonde de détection), on utilisera par exemple en combinaison une sonde spécifique de la famille des spirochètes et une sonde spécifique de l'espèce considérée. On peut aussi utiliser en combinaison deux sondes spécifiques de ladite espèce, lorsqu'elles existent, ces deux sondes étant complémentaires de régions non chevauchantes du gène *rpoB.*

Une autre application d'un oligonucléotide de l'invention est son utilisation comme amorce nucléotidique comprenant un oligonucléotide monocaténaire choisi parmi les oligonucléotides ayant une séquence d'au moins 12 motifs nucléotidiques incluse dans l'une des séquences SEQ ID N°1 à 4, qui est utilisable dans la synthèse d'un acide nucléique en présence d'une polymérase par un procédé connu en soi, notamment dans des méthodes d'amplification utilisant une telle synthèse en présence d'une polymérase (PCR, RT-PCR, etc.). En particulier, une amorce de l'invention peut être utilisée pour la transcription inverse spécifique d'une séquence d'ARN messager d'au moins une espèce ou d'au moins un groupe d'espèces de spirochètes pour obtenir une séquence d'ADN complémentaire correspondante. Une telle transcription inverse peut constituer le premier stade de la technique RT-PCR, le stade suivant étant l'amplification par PCR de l'ADN complémentaire obtenu. On peut également utiliser les amorces de l'invention pour l'amplification spécifique par réaction de polymérisation en chaîne de la séquence de l'ADN du gène *rpoB* d'au moins une espèce ou d'au moins un groupe d'espèces de spirochètes.

Selon un cas particulier, ladite amorce comprenant un oligonucléotide de l'invention comprend en outre la séquence sens ou anti-sens d'un promoteur reconnu par une ARN polymérase (promoteurs T7, T3, SP6 par exemple [Studier FW, BA Moffatt (1986) J. Mol. Biol. 189:113] : de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7^{th} International Conference on AIDS (1991) Florence, Italy]

Un autre objet de l'invention est une amorce nucléotidique comprenant un oligonucléotide monocaténaire choisi parmi les oligonucléotides ayant une séquence d'au moins 12 motifs nucléotidiques consécutifs incluse dans l'une des séquences SEQ ID n°1 à SEQ ID N°4 qui est utilisable pour le séquençage total ou partiel du gène *rpoB* chez une quelconque des espèces de spirochètes. En particulier, l'amorce nucléotidique est utilisable pour le séquençage d'un acide nucléique amplifié.

En effet, les amorces oligonucléotidiques objets de l'invention permettent l'amplification puis le séquençage du gène *rpoB* chez tout spirochète, et l'identification de tout spirochète par analyse bio-informatique de cette séquence et la reconnaissance de nouvelles espèces de spirochètes inconnues. Le séquençage est l'obtention de la séquence totale ou partielle du gène *ropB* par un procédé connu en soi, polymérisation absortive utilisant des di-déoxynucléotides [Sanger F., Coulson A.R. (1975) J. Mol. Biol. 94:441] ou hybridations multiples utilisant les puces à ADN.

Les amorces n° 1 à n° 3 peuvent être utilisées comme amorces 5' et les amorces n° 2 à n° 4 comme amorces 3' pour amplifier un fragment de gène *rpoB* encadré en 5' et 3' par lesdites amorces.

Dans un mode de réalisation, on utilise donc un jeu de deux amorces consistant dans deux oligonucléotides différentes choisis parmi les oligonucléotides ayant une séquence d'au moins 12 motifs nucléotidiques incluses dans les séquences SEQ ID N° 1 à 3 pour l'amorce 5' et respectivement SEQ ID N° 2 à 4 pour l'amorce 3'.

La présente invention a donc également pour objet un procédé, caractérisé en ce qu'on amplifie tout d'abord le fragment du gène *rpoB* de ladite bactérie avec des amorces selon l'invention, puis on met en contact ledit fragment avec une sonde de ladite bactérie selon l'invention, et on détermine la formation ou l'absence de formation d'un complexe d'hybridation entre ladite sonde et ledit fragment.

La présente invention a également pour objet un procédé de détermination de la présence ou de l'absence d'au moins une bactérie de l'ordre des *spirochaetales* dans un échantillon contenant ou susceptible de contenir des acides nucléiques d'au moins une telle bactérie, caractérisé en ce qu'on met en contact ledit échantillon avec des amorces selon l'invention, puis on réalise une amplification et on détermine l'apparition ou l'absence d'un produit d'amplification.

Dans un mode de réalisation, un procédé de détermination de la présence ou de l'absence d'au moins une bactérie de l'ordre des *spirochaetales* dans un échantillon biologique contenant ou susceptible de contenir des acides nucléiques de ladite bactérie, est caractérisé en ce qu'on amplifie et on réalise le séquençage d'un fragment du gène *rpoB* de ladite bactérie dans ledit échantillon à l'aide d'amorces selon l'invention et on compare la séquence du fragment du gène *rpoB* du gène obtenu avec la séquence connue du fragment du gène *rpoB* de ladite bactérie, et on détermine ainsi la présence de ladite bactérie dans l'échantillon si la séquence du fragment obtenu est identique à la séquence connue du fragment du gène *rpoB* de ladite bactérie.

La présente invention a également pour objet une sonde de détection, dans un échantillon biologique, spécifique d'une espèce de bactérie appartenant à l'ordre des *spirochaetales,* caractérisée en ce qu'elle comprend un fragment du gène *ropB* pouvant être obtenu par amplification à l'aide d'amorces selon l'invention, de préférence avec l'une des séquences SEQ ID n° 1 à 3 comme amorce 5' et l'une des séquences SEQ ID n° 2 à 4 comme amorce 3'. Ledit fragment du gène *rpoB* encadré par les oligonucléotides selon l'invention correspond en effet à une zone très variable selon les espèces desdites bactéries appartenant à l'ordre des *spirochaetales* et spécifique pour chaque dite espèce.

Enfin, un dernier objet de l'invention est une sonde de thérapie génique pour traiter les infections provoquées par au moins une espèce ou un groupe d'espèces de spirochètes, ladite sonde comprenant un oligonucléotide tel que défini précédemment. Cette sonde de thérapie génique, capable de s'hybrider sur l'ARN messager et/ou sur l'ADN génomique desdites bactéries, peut bloquer les phénomènes de traduction et/ou de transcription et/ou de réplication.

Le principe des méthodes de thérapie génique est connu et repose notamment sur l'utilisation d'une sonde correspondant à un brin anti-sens : la formation d'un hybride entre la sonde et le brin sens est capable de perturber au moins l'une des étapes du décryptage de l'information génétique. Les sondes de thérapie génique sont donc utilisables comme médicaments antibactériens, permettant de lutter contre les infections causées par des spirochètes.

L'invention va maintenant être décrite en détail à l'aide de l'exposé expérimental ci-après.

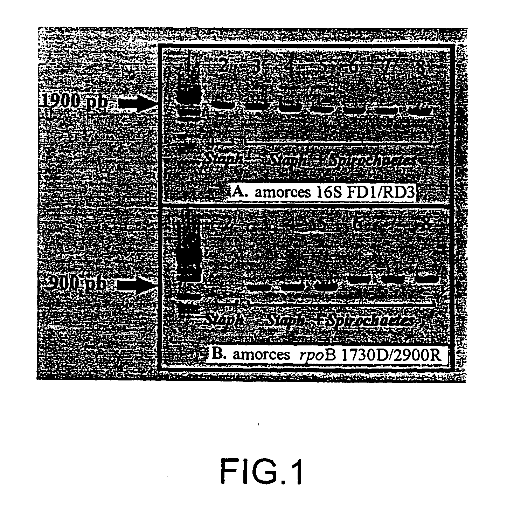

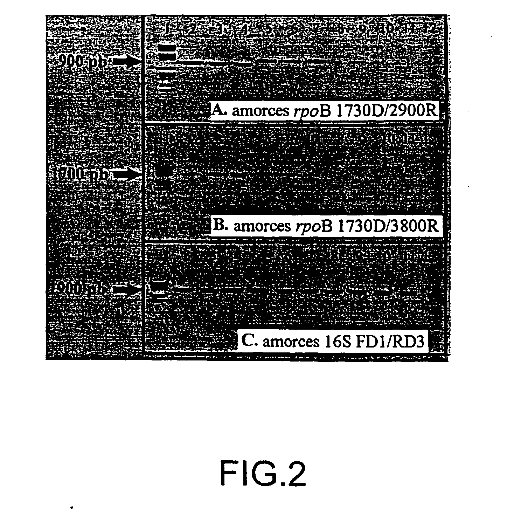

La description ci-après sera mieux comprise à l'aide des figures 1 et 2 sur lesquelles :
- la figure 1 est une photographie d'un gel d'électrophorèse mettant en évidence la détection des spirochètes à partir d'une suspension mixte bactérienne en utilisant les sondes de l'invention; et
- la figure 2 est une photographie d'un gel d'électrophorèse mettant en évidence l'amplification spécifique par PCR de fragments du gène *rpoB* des spirochètes en utilisant les amorces de l'invention.

Les souches de spirochètes utilisées dans les exemples ci-après, à savoir *Borrelia burgdorferi, Borrelia recurrentis, Treponema pallidum, Leptospira biflexa serovar patoc* (appelée ci-après *Leptospira biflexa), Leptospira interrogans serovar icterohaemmorragiae* (appelée ci-après *Leptospira icterohaemmorragiae) et Leptospira interrogans serovar australis* (appelée ci-après *Leptospira australis),* ont toutes été obtenues de la collection ATCC, à l'exception de *Borrelia recurrentis* qui est disponible auprès du Centre National de référence, Institut Pasteur Paris (France). Le numéro ATCC de *Borrelia burgdorferi* est 35210, celui de *Treponema Pallidum* 27087, celui de *Leptospira biflexa* 23582, celui de *Leptospira icterohaemmorragiae* 43642 et celui de *Leptospira australis* 23605.

Les souches *Borrelia et Leptospira* ont été cultivées à 30°C sur les milieux BSKII et EMJH, respectivement [Barbour A.G. (1984) Yale J. Biol. Med. 57:521]. Comme *T. pallidum* ne peut pas être cultivée *in vitro,* cette bactérie pathogène a été propagée par injections dans les testicules d'un lapin.

Les autres souches de bactéries utilisées, *Escherichia coli, Staphylococcus aureus, Streptococcus salivarius* et *Pseudomonas aeruginosa,* ont été isolées cliniquement de patients hospitalisés à Marseille.

### EXEMPLE 1 : Détection spécifique des spirochètes à l'aide des sondes de l'invention

Cette expérience a été réalisée avec les souches de spirochètes suivantes : *Borrelia burdorferi, Treponema pallidum, Leptospira biflexa, Leptospira. Icterohaemmorragiae, Leptospira australis et Borrelia recurrentis,* ainsi qu'avec *Staphylococcus aureus.*

On a préparé une suspension bactérienne mixte en mélangeant un ADN de spirochète avec un extrait de *Staphylococcus aureus.*

On a réalisé une méthode PCR en utilisant la trousse QIAamp tissue kit (Qiagen) et la Taq Polymérase de Gibco (Gibco BRL, USA). Après une première étape de dénaturation (94°C pendant 2 min), on a répété 35 fois un cycle de 3 étapes à 94°C pendant 30 s, 52°C pendant 30 s et 72°C pendant 1 min. On a terminé le programme PCR, on a purifié et séquencé les amplicons résultants comme indiqué précédemment.

Les résultats sont indiqués sur la figure 1, sur laquelle
la bande 1 correspond aux marqueurs de masse moléculaire (Boehringer),
la bande 2 correspond à *S*. *aureus* seul,
la bande 3 correspond à *B. burgdorferi* + *S. aureus,*
la bande 4 correspond à *B. recurrentis* + *S. aureus,*
la bande 5 correspond à *T. pallidum* + *S. aureus,*
la bande 6 correspond à *L. biflexa* + *S. aureus,*
la bande 7 correspond à *L. australis* + *S. aureus,*
la bande 8 correspond à *L. icterohaemmorragiae* + *S. aureus.*

Les amorces utilisées étaient :
panneau A : les amorces d'ARNr 16S FD1 et RD3 [Weisburg et al. J. Bacteriol. (1991) 173:697-703]
panneau B : les amorces 1730D et 2900R de l'invention.

L'amorce 1730D a la séquence SEQ ID N° 1 (5'CTTGGNCCNGGNGGACTTTC3') dans laquelle "N" est l'inosine et l'amorce 2900R a la séquence SEQ ID N°2 (5'AGAAATNAANATNGCATCCTC3') dans laquelle "N" est l'inosine.

Les résultats montrent que les amorces de l'invention n'ont pas été capables d'amplifier l'ADN de *S. aureus* seul, et donc de détecter *S. aureus,* mais ont été capables d'amplifier celui des spirochètes, et donc de le détecter, même en présence de *S. aureus* (panneau B), contrairement aux amorces 16S qui ont été capables d'amplifier l'ADN de *S. aureus* seul (panneau A).

### EXEMPLE 2 : Amplification spécifique de fragments de gène rpoB à l'aide des amorces de l'invention

Cette expérience a été réalisée avec les souches de spirochètes suivantes : *Borrelia burdorferi, Treponema pallidum, Leptospira biflexa, Leptospira icterohaemmorragiae, Leptospira australis et Borrelia recurrentis,* ainsi qu'avec les autres souches non spirochètes *Escherichia coli,* Staphylococcus *aureus, Streptococcus salivarius* et *Pseudomonas aeruginosa.*

La séquence du gène *rpoB* de *Treponema pallidum* utilisée est celle décrite par Weinstock et al. [(1998) The genome of *Treponema pallidum* : new light on the agent of syphilis. *FEM Microbiol. Rev.* 22:323-332].

La séquence du gène *rpoB* de *Borrelia burgdorferi* utilisée est celle décrite par Alekshun et al. [(1997) Molecular cloning and characterization of *Borrelia burgdorferi* rpoB. *Gene* 186:227-235].

Les séquences d'ADN du gène *rpoB* de *Leptospira biflexa,* L*eptospira icterohaemmorragiae, Leptospira australis et Borrelia recurrentis* ont été obtenues par PCR de la façon suivante :

L'ADN génomique de *Leptospira biflexa* a été extrait en suivant les procédures standards au phénol/chloroforme [Sambrook et al. (1989) Molecular Cloning : A Laboratory Manual. Cold Spring Harbor, NY]. Dans cette première étape d'expériences, on a réalisé les amplifications par PCR avec les amorces SEB1 (5'-AAC CAG TTC CGC GTT GGC CTG G-3') et SEB2 (5'-CCT GAA CAA CAC GCT CGG A-3') (SEB pour *Staphylococcus aureus, Escherichia coli et Borrelia subtilis)* et la *Taq* ADN polymérase de Eurogentec (de la société Seraing, Belgique) en utilisant 5 µl d'ADN pour un volume final de 50 µl. Après une première étape de dénaturation (95°C pendant 1,5 min), on a répété 35 fois un cycle à 3 étapes de 95°C pendant 20 s, 50°C pendant 30 s et 72°C pendant 1 min. On a terminé le programme PCR par une seule étape d'extension de 3 min à 72°C (modèle de cycle thermique Peltier PTC 200, MJ Research, Watertown, MA, USA).

On a ensuite séparé les amplicons obtenus par électrophorèse sur gel d'agarose à 1% et on les a visualisés par coloration avec du bromure d'éthidium.

On a réalisé le séquençage du gène par purification des échantillons avec le kit de purification PCR QIAquick (Qiagen, Hilden, Allemagne) et on les a mis à réagir avec le tampon de réaction dRhodamine Terminator Cycle Sequencing Ready Reaction buffer (kit de séquençage d'ADN, Perkin-Elmer). Enfin, on a effectué une électrophorèse des produits réactionnels avec le séquenceur d'ADN automatique Applied Biosystems model ABI 310 (Perkin-Elmer).

La séquence de l'extrémité 5' du gène *rpoB* a été obtenue en utilisant la trousse Universal Genome Walker^{™} (Clontech, Palo Alto, CA, USA) de la façon suivante. Cinq regroupements de fragments d'ADN génomique appelés "banques" Genome Walker ont été construits en utilisant les enzymes de restriction *Dra*I*, EcoR*V*, Pvu*II, *Sca*I et *Stu*I*.* Ces banques ont été utilisées pour un séquençage en amont de l'ADN génomique en utilisant, d'une part, une amorce spécifique du gène dont la séquence correspond à la région aussi proche que possible de l'extrémité 5' connue du gène et, d'autre part, une amorce d'adaptation fournie dans la trousse.

On a purifié les fragments amplifiés et on a utilisé une source d'ADN pour une deuxième PCR emboîtée. On a ensuite traité les amplicons obtenus comme décrit précédemment pour le séquençage automatisé.

Le gène *rpoB* de *Leptospira biflexa* obtenu a la séquence SEQ ID N°5 et possède 3876 acides nucléiques. Le gène *rpoB* de *Leptospira icterohaemorragiae.* obtenu a la séquence SEQ ID N°6 et possède 914 acides nucléiques. Le gène *rpoB* de *Leptospira australis* obtenu a la séquence SEQ ID N°7 et possède 949 acides nucléiques. Enfin, le gène *rpoB* de *Borrelia recurrentis* obtenu a la séquence SEQ ID N°8 et possède 800 acides nucléiques.

Les séquences d'ADN du gène *rpoB* de *Escherichia* coli, *Staphylococcus aureus, Streptococcus salivarius* et *Pseudomonas aeruginosa* sont décrites dans la littérature (Gen Bank).

Les fragments du gène *rpoB* à amplifier étaient constitués de 949 pb.

Les amplifications par PCR des fragments du gène *rpoB* ont été réalisées comme dans l'exemple 3, à ceci près qu'on a utilisé chaque espèce séparément.

Les résultats sont indiqués sur la figure 2, sur laquelle :
la bande 1 correspond aux marqueurs de masse moléculaire (Boehringer),
la bande 2 correspond à *B. burgdorferi,*
la bande 3 correspond à *B. recurrentis,*
la bande 4 correspond à *T. pallidum,*
la bande 5 correspond à *L. biflexa,*
la bande 6 correspond à *L. australis,*
la bande 7 correspond à *L. icterohaemmorragiae,*
la bande 8 correspond à *E*. *coli,*
la bande 9 correspond à *S*. *aureus,*
la bande 10 correspond à *Str. salivarius,*
la bande 11 correspond à *Ps. aeruginosa,*
la bande 12 correspond à un témoin négatif sans ADN.

Les amorces utilisées étaient :
panneau A : l'amorce 1730D et l'amorce 2900R de l'invention,
panneau B : l'amorce 1730D et l'amorce 3800R de l'invention et
panneau C : les amorces d'ARNr 16S RD1 et FD3.

L'amorce 3800R a la séquence SEQ ID N°4 (5'GCTTCNAGNGCCCANAC3') dans laquelle "N" est l'inosine.

Les résultats montrent que les amorces de l'invention ont permis uniquement l'amplification des fragments de l'ADN des spirochètes (panneaux A et B), tandis que les amorces 16S ont tout amplifié (panneau C).

L'amorce SEQ ID n° 3 (5'GGGTGNATTTTNTCATCNAC3') peut aussi être utilisée comme amorce. Les amorces n° 1 et 3 selon l'invention ont permis par amplification puis séquençage du produit amplifié la détection et l'identification de la bactérie *Borrelia duttonii* dans une tique africaine collectée qui ZAÏRE de l'espèce *ornithodoros moubata* et le sang total d'un patient zaïrois présentant une fièvre récurrente.

## Revendications

1. Oligonucléotides monocaténaires choisis parmi les oligonucléotides comprenant une séquence d'au moins 12 motifs nucléotidiques consécutifs incluse dans l'une des séquences SEQ ID N°1 à SEQ ID N°4 dans lesquelles « N » représente un nucléotide identique ou différent choisi parmi l'inosine ou un mélange équimolaire de 4 nucléotides différents choisis parmi A, T, C et G, et parmi les oligonucléotides complémentaires de ces oligonucléotides.

2. Oligonucléotides selon la revendication 1, **caractérisés en ce qu'**ils comprennent de 12 à 35 motifs nucléotidiques.

3. Oligonucléotides selon la revendication 2, **caractérisés en ce qu'**ils ont une séquence choisie parmi les séquences SEQ ID N° 1 à 4 et les séquences complémentaires.

4. Oligonucléotides selon l'une des revendications 1 à 3, **caractérisés en ce que** « N » représente l'inosine.

5. Oligonucléotides selon l'une des revendications 1 à 3, **caractérisés en ce qu'**ils comprennent un mélange l'oligonucléotides comprenant des séquences incluses dans l'une des séquences SEQ ID n° 1 à 4 dans lesquelles tous les nucléotides A, T, C et G sont représentés à chacune des positions où « N » apparaît.

6. Sonde pour la détection, dans un échantillon biologique, de bactéries appartenant à l'ordre des *Spirochaetales* **caractérisée en ce qu'**elle comprend un oligonucléotide selon l'une des revendications 1 à 5.

7. Sonde selon la revendication 6, **caractérisée en ce qu'**elle est immobilisée sur un support solide.

8. Sonde selon la revendication 7, **caractérisée en ce qu'**elle est marquée avec un agent traceur.

9. Procédé de détermination de la présence ou de l'absence d'au moins une bactérie appartenant à l'ordre des *spirochaetales* dans un échantillon contenant ou susceptible de contenir des acides nucléiques d'au moins une telle bactérie, **caractérisé en ce qu'**on met en contact ledit échantillon avec au moins une sonde selon l'une quelconque des revendications 6 à 8, puis on détermine la formation ou l'absence de formation d'un complexe d'hybridation entre ladite sonde et l'acide nucléique de l'échantillon.

10. Amorce nucléotidique utilisable pour la synthèse en présence d'une polymérase, et le séquençage total ou partiel du gène *rpoB* chez l'une quelconque des espèces de bactéries appartenant à l'ordre des *spirochaetales,* **caractérisée en ce qu'**elle comprend un oligonucléotide selon l'une des revendications 1 à 5.

11. Procédé selon la revendication 9, **caractérisé en ce qu'**on réalise une amplification d'un fragment du gène *rpoB* de ladite bactérie, avec au moins une amorce selon la revendication 10, puis on met en contact ledit fragment avec une sonde de ladite bactérie selon l'une des revendications 6 à 8, et on détermine la formation ou l'absence de formation d'un complexe d'hybridation entre ladite sonde et ledit fragment.

12. Procédé de détermination de la présence ou de l'absence d'au moins une bactérie appartenant à l'ordre des *spirochaetales* dans un échantillon contenant ou susceptible de contenir des acides nucléiques d'au moins une telle bactérie, **caractérisé en ce qu'**on met en contact ledit échantillon avec des amorces selon l'une la revendication 10, puis on réalise une amplification et on détermine l'apparition ou l'absence d'un produit d'amplification.

13. Procédé selon la revendication 12, **caractérisé en ce qu'**on réalise le séquençage du fragment du gène *rpoB* amplifié et on compare la séquence dudit fragment obtenu avec la séquence connue du gène *rpoB* de ladite bactérie, et on détermine ainsi la présence de l'espèce de ladite bactérie si le séquençage dudit fragment obtenu est identique à ladite séquence connue.

14. Sonde de détection dans un échantillon biologique, spécifique d'une espèce de bactérie appartenant à l'ordre des *spirochaetales,* **caractérisée en ce qu'**elle comprend un fragment du gène *ropB* pouvant être obtenu par amplification à l'aide d'amorce selon la revendication 10.

15. Sonde de thérapie génique, **caractérisée en ce qu'**elle comprend un oligonucléotide selon l'une des revendications 1 à 5.

## Claims

1. Single stranded oligonucleotides chosen from among the oligonucleotides comprising a sequence of at least 12 consecutive nucleotide motifs included in one of the sequences SEQ ID N° 1 to SEQ ID N° 4 in which "N" represents an identical or different nucleotide chosen from among inosine or an equimolar mixture of 4 different nucleotides chosen from among A, T, C or G and among the complementary oligonucleotides of these oligonucleotides.

2. Oligonucleotides according to claim 1, **characterised in that** they contain 12 to 35 nucleotide motifs.

3. Oligonucleotides according to claim 2, **characterised in that** they have a sequence chosen from among sequences SEQ ID N° 1 to 4 and the complementary sequences.

4. Oligonucleotides according to one of claims 1 to 3, **characterised in that** "N" represents inosine.

5. Oligonucleotides according to one of claims 1 to 3, **characterised in that** they comprise a mixture of oligonucleotides comprising sequences included in one of sequences SEQ ID N° 1 to 4 in which all of the nucleotides A, T, C and G are represented at each of the positions where "N" appears.

6. Probe for the detection, in a biological sample, of bacteria belonging to the order of *Spirochaetales* **characterised in that** it includes a nucleotide according to one of claims 1 to 5.

7. Probe according to claim 6, **characterised in that** it is immobilised on a solid support.

8. Probe according to claim 7, **characterised in that** it is marked with a tracing agent.

9. Method to determine whether at least one bacteria belonging to the order of *Spirochaetales* is present in a sample containing or likely to contain nucleic acids from at least one such bacteria, **characterised in that** the said sample is put into contact with at least one probe according to any one of claims 6 to 8, then to determine whether a hybridation complex is formed between the said probe and the nucleic acid in the sample.

10. Nucleotide primer that can be used for the synthesis in the presence of a polymerase, and the total or partial sequencing of gene *rpoB* in any one of the species of bacteria belonging to the order of *Spirochaetales,* **characterised in that** it includes an oligonucleotide according to one of claims 1 to 5.

11. Method according to claim 9, **characterised in that** a fragment of gene *rpoB* of the said bacteria is amplified with at least one primer according to claim 10. The said fragment is then put into contact with a probe of the said bacteria according to one of claims 6 to 8, and whether a hybridation complex is formed between the said probe and the said fragment is determined.

12. Method to determine whether at least one bacteria belonging to the order of *Spriochaetales* is present in a sample containing or likely to contain nucleic acids from at least one such bacteria, **characterised in that** the said sample is put into contact with the primers according to claim 10. An amplification is then carried out and the presence or absence of an amplification product is determined.

13. Method according to claim 12, **characterised in that** the sequencing of a amplified fragment of gene *rpoB* is carried out and the sequence of the said fragment obtained is compared with the known sequence of gene *rpoB of* the said bacteria. The presence of the species of the said bacteria is determined if the sequencing of the said fragment obtained is identical to that of the known sequence.

14. Detection probe in a biological sample, specific for a species of bacteria belonging to the order of *Spirochaetales,* **characterised in that** it includes a fragment of gene *rpoB* that can be obtained by amplification by means of a primer according to claim 10.

15. Gene therapy probe, **characterised in that** it includes an oligonucleotide according to one of claims 1 to 5.

## Patentansprüche

1. Einzelsträngige Oligonukleotide, gewählt unter den Oligonukleotiden, die eine Sequenz mit wenigstens zwölf aufeinanderfolgenden Nukleotidmotiven umfassen, eingeschlossen in einer der Sequenzen SEQ ID Nr. 1 bis SEQ ID Nr. 4, in denen "N" ein identisches oder unterschiedliches Nukleotid darstellt, gewählt unter Inosin oder einem äquimolaren Gemisch von vier unterschiedlichen Nukleotiden, gewählt unter A, T, C und G und unter den komplementären Oligonukleotiden dieser Oligonukleotide.

2. Oligonukleotide nach Anspruch 1, **dadurch gekennzeichnet, daß** sie 12 bis 35 Nukleotidmotive umfassen.

3. Oligonukleotide nach Anspruch 2, **dadurch gekennzeichnet, daß** sie eine Sequenz haben, die gewählt ist unter den Sequenzen SEQ ID Nr. 1 bis 4 und den komplementären Sequenzen.

4. Oligonukleotide nach einem der Ansprüche 1 bis 3, **dadurch gekennzeichnet, daß** "N" Inosin darstellt.

5. Oligonukleotide nach einem der Ansprüche 1 bis 3, **dadurch gekennzeichnet, daß** sie ein Gemisch von Oligonukleotiden umfassen, umfassend Sequenzen, die in einer der Sequenzen SEQ ID Nr. 1 bis 4 eingeschlossen sind, in denen alle Nukleotide A, T, C und G an jeder der Positionen dargestellt sind, wo "N" erscheint.

6. Sonde zur Detektion, in einer biologischen Probe, von Bakterien, die zur Ordnung der *Spirochaetales* gehören, **dadurch gekennzeichnet, daß** sie ein Oligonukleotid nach einem der Ansprüche 1 bis 5 umfaßt.

7. Sonde nach Anspruch 6, **dadurch gekennzeichnet, daß** sie auf einem festen Träger immobilisiert ist.

8. Sonde nach Anspruch 7, **dadurch gekennzeichnet, daß** sie mit einem Tracerreagenz markiert ist.

9. Verfahren zur Bestimmung der Gegenwart oder der Abwesenheit wenigstens eines Bakteriums, das zur Ordnung der *Spirochaetales* gehört in einer Probe, die Nukleinsäuren von wenigstens einem solchen Bakterium enthält oder geeignet ist, sie zu enthalten, **dadurch gekennzeichnet, daß** die Probe mit wenigstens einer Sonde nach einem der Ansprüche 6 bis 8 kontaktiert wird, und dann die Bildung oder Abwesenheit von Bildung eines Hybridisierungskomplexes zwischen der Sonde und der Nukleinsäure der Probe bestimmt wird.

10. Nukleotidprimer, verwendbar für die Synthese in Gegenwart einer Polymerase und die vollständige oder teilweise Sequenzierung des Gens rpoB, bei irgendeiner der Bakterienspezies, die zur Ordnung der *Spirochaetales* gehören, **dadurch gekennzeichnet, daß** er ein Oligonukleotid nach einem der Ansprüche 1 bis 5 umfaßt.

11. Verfahren nach Anspruch 9, **dadurch gekennzeichnet, daß** man eine Amplifikation eines Genfragments rpoB des Bakteriums durchführt mit wenigstens einem Primer nach Anspruch 10 und dann das Fragment mit einer Sonde des Bakteriums nach einem der Ansprüche 6 bis 8 kontaktiert und man die Bildung oder Abwesenheit einer Bildung eines Hybrisierungskomplexes zwischen der Sonde und dem Fragment bestimmt.

12. Verfahren zur Bestimmung der Gegenwart oder Abwesenheit wenigstens eines Bakteriums, das zur Ordnung der *Spirochaetales* gehört, in einer Probe, die Nukleinsäuren von wenigstens einem solchen Bakterium enthält oder geeignet ist, sie zu enthalten, **dadurch gekennzeichnet, daß** man die Probe mit Primern nach Anspruch 10 kontaktiert und man dann eine Amplifikation durchführt und man das Auftreten oder die Abwesenheit eines Amplifikationsproduktes bestimmt.

13. Verfahren nach Anspruch 12, **dadurch gekennzeichnet, daß** man das Sequenzieren des amplifizierten Fragments des Gens rpoB durchführt und man die Sequenz des Fragments, das mit der bekannten Sequenz des Gens rpoB des Bakteriums erhalten wird, vergleicht und man so die Gegenwart der Spezies des Bakteriums bestimmt, wenn die Sequenzierung des erhaltenen Fragments identisch mit der bekannten Sequenz ist.

14. Sonde zur Detektion in einer biologischen Probe, spezifisch für eine Spezies eines Bakteriums, das zur Ordnung der *Spirochaetales* gehört, **dadurch gekennzeichnet, daß** sie ein Fragment des Gens rpoB enthält, das erhalten werden kann durch Amplifikation mit Hilfe des Primern nach Anspruch 10.

15. Sonde zur Gentherapie, **dadurch gekennzeichnet, daß** sie ein Oligonukleotid nach einem der Ansprüche 1 bis 5 enthält.


### LISTAGE DE SEQUENCES

<110> Université de la Méditerranée (Aix-Marseille II)
   <120> Oligonucléotides monocaténaires, sondes, amorces et procédé de détection des spirochètes
<160> 8
<170> PatentIn Ver. 2.1
<210> 1
   <211> 20
   <212> ADN
   <213> Séquence artificielle
<220>
   <223> Description de la séquence artificielle : amorce n°1
<400> 1
   cttggnccng gnggactttc 20
<210> 2
   <211> 21
   <212> ADN
   <213> Séquence artificielle
<220>
   <223> Description de la séquence artificielle : amorce n°2
<400> 2
   agaaatnaan atngcatcct c 21 <210> 3
   <211> 20
   <212> ADN
   <213> Séquence artificielle
<220>
   <223> Description de la séquence artificielle : amorce n°3
<400> 3
   gggtgnattt tntcatcnac 20 <210> 4
   <211> 17
   <212> ADN
   <213> Séquence artificielle
<220>
   <223> Description de la séquence artificielle : amorce n°4
<400> 4
   gcttcnagng cccanac 17 <210> 5
   <211> 3876
   <212> ADN
   <213> Leptospira biflexa
<400> 5
   gctgatatta agaaaaaact ncgaaggtnt tgggnctcaa gtagaagttg ctggctgccc 60
<210> 6
   <211> 914
   <212> ADN
   <213> Leptospira icterohaemorragiae
<400> 6
   ttcactattc tcactacggt agaatgtgtc cgattgaaac tccggaaggt ccgaacatcg 60
   gtctgattct ttccatgtct tcttacgctc gtgtgaatga ctacggattc ttggaaactc
   120
   cttacagaac cgtgaagaac ggtaaagtta ccggtcagat cgagcacctt accgcagaca
   180
   aagaagaata tcattacatc gctcaagctt ccggcgtgat cgatgaaaaa ggcgagctca
   240
   aaaacaaatt gatttccacg cgtcacagag gggatttccc tttccgtaac ccgagcgaga
   300
   ttcagtatat ggacttggck cctctacaag tcgtttcggt ttccacggcg ctgattccgt
   360
   tccttgaaca cgacgacgcg aaccgcgcct catgggttcc aacatgcaac gtcaggcggt
   420
   tcctcttctc cgtgaagaag ctcttttgta ggaactggta tggaaaccag agccgcttac
   480
   gattccagaa tttgtatcgt aaacaaacac gacggtgtcg taacttccgt cgatgcggaa
   540
   aacatcgttg tagaaagaaa gggcggaaaa gaatccgata cgtatcaact tacgaaattc
   600
   aaaaagacaa accaaggaac tgctttaatc agaagccgat tgtaggagtg gttcactccg
   660
   agatcaatgg aaaggtttcc aaggtttcca aagaaaaaat cgaagtcact ggtgaaaacg
   720
   gtgaactgaa agaatatgtt cttcaaatcg gaagcaaaca atattctccg atcgtctctg
   780
   caggcgaaga agtaaaaaga ggatcgactc tcgcaggaca agttgttgta ggtgagaagt
   840
   tggatgagat gggaaatatc ctcgtaaaag gaaccgttct tgctgatggt cctgcggtcg
   900
   acaacggagt tctc
   914
<210> 7
   <211> 949
   <212> ADN
   <213> Leptospira australis
<400> 7
   gtgacgttca ctattctcac tacggtagaa tgtgtccgat tgaaactccg gaaggtccga 60
   acatcggtct gattctttcc atgtcttctt acgctcgtgt gaatgactac ggattcttgg 120
   aaactcctta cagaaccgtg aagaacggta aagttaccgg tcagatcgag caccttaccg 180
   cagacaaaga agaatatcat tacatcgctc aagcttccgg cgtgatcgat gaaaaaggcg 240
   agctcaaaaa caaattgatt tccacgcgtc acagagggga tttccctttc cgtaacccga 300
   gcgagattca gtatatggac ttggctcctc tacaagtcgt ttcggtttcc acggcgctga 360
   ttccgttcct tgaacacgac gacgcgaacc gcgccctcat gggttccaac atgcaacgtc 420
   aggcggttcc tcttcttcgt gaagaagctc cttttgtcgg aaccggtatg gaaaccagag 480
   ccgcttacga ttccagaatt tgtatcgtaa acaaacacga cggtgtcgta acttccgtcg 540
   atgcggaaaa catcgttgta gaaagaaagg gcggaaaaga atccgatacg tatcaactta 600
   cgaaattcaa aaagacaaac caaggaacct gctttaatca gaagccgatt gtaggagtgg 660
   ttcactccga gattaacgga aaggtttcca aggtctccaa agaaaaaatc gaagtcactg 720
   gtgaaaacgg tgaactaaaa gaatatgttc ttcaaatcgg aagcaaacaa tattctccga 780
   tcgtctccgc aggcgaagaa gtaaaacgag gatcgactct cgcaggacaa gttgttgtag 840
   gtgagaagtt ggatgagatg ggaaatatcc tcgtaaaagg aaccgttctt gctgatggtc 900
   ctgcggtcga caacggagtt ctcgctctgg gaagaaacgt tctcgcggc 949
<210> 8
   <211> 800
   <212> ADN
   <213> Borrelia recurrentis
<400> 8
   aagggatcga gcagctttga agtaagatat gtacattata cccattatgg taggatgtgt 60
   cctattgaaa ctcctgaagg cccaaatatt ggacttattg tttctttggc tacttattca
   120
   aaagttaatg attatggttt cttagaaact ccttatagga aggtgattga tggtaaggtg
   180
   accgatgata ttgaatattt gtctgctatt gatgaggaaa aaaaatgtat tgcgcaagca
   240
   aatgcttctg ttagttctga tggtaattat actgatgatt tggtgtctgt taggatttct
   300
   ggggattata ctacaatgat gcctaaaaat atcgattaca tggatgtttc gcctagacaa
   360
   ttaatatctg tctcttcggc gttaatactt ttcttgaaca taatgatgca aatcgtgctc
   420
   ttatgggttc gaatatgcaa cgtcaggcag ttcttattat ttccacagcc acctattgtt
   480
   ggtacaggta tggagaggat agttgcaaaa gactctggtg ttgttattaa agcaaaaaga
   540
   cctggtagag ttgtcttagc cacaaacaaa aagatagtta ttaaacctga taatgcaact
   600
   tctgaacgag atttagatga atatgaactt tataaatatg agaggacaaa ccaggatact
   660
   tctttcaatc attcagtttt ggtgaagaat ggccaaattg ttaataagga tgagataata
   720
   gcagatggtc ctgctactag atatggagaa ttggcgcttg gtaataattt attagttgtt
   780
   ttattccgtg gaatggattt
   800